# EUROPÄISCHE PATENTSCHRIFT

(11) **EP 0 639 952 B1**
(45) Veröffentlichungstag und Bekanntmachung des Hinweises auf die Patenterteilung: **28.05.1997**
(21) Anmeldenummer: 93914741.9
(22) Anmeldetag: 29.06.1993
(51) Int. Cl.: A61B 5/117

(54) **VERFAHREN UND VORRICHTUNG ZUR IDENTIFIKATION VON PRÜFOBJEKTEN**
PROCESS AND DEVICE FOR IDENTIFYING OBJECTS TO BE INSPECTED
PROCEDE ET DISPOSITIF D'IDENTIFICATION D'OBJETS A CONTROLER

(30) Priorität: 08.07.1992 DE 4222387
(43) Veröffentlichungstag der Anmeldung: 01.03.1995
(73) Patentinhaber: Stockburger, Hermann, 79410 Badenweiler (DE)
(72) Erfinder: Stockburger, Hermann, 79410 Badenweiler (DE)
(74) Vertreter: Schmitt, Hans, Dipl.-Ing.
(86) Internationale Anmeldenummer: EP9301659
(87) Internationale Veröffentlichungsnummer: WO9401043

(56) Entgegenhaltungen:
- EP-A- 0 048 489
- EP-A- 0 052 349
- EP-A- 0 402 779
- WO-A-92/09049

## Beschreibung

Die Erfindung betrifft ein Verfahren zur Bestimmung der Echtheit und/oder Identifikation der jeweiligen Raumstruktur eines Prüfobjektes insbesondere der Epithelstruktur der Haut z.B. eines menschlichen Fingers, wobei dieses Prüfobjekt auf eine ebene oder gewölbte Fläche aufgelegt und mit durch einen an diese Fläche angrenzenden Körper verlaufenden Ultraschallwellen beschallt wird und die am Prüfobjekt reflektierten und/oder rückgestreuten Ultraschallwellen eine jeweils verschiedene Struktur unterschiedlicher Prüfobjekte wiedergeben und ausgewertet werden.

Ein derartiges Verfahren ist aus der DE-OS 40 16 105 bekannt. Eine eindeutige Identifikation einzelner menschlicher Individuen ist möglich, da die Epithelstrukturen durch den speziellen genetischen Code und die individuellen Wachstumsphasen signifikant von Mensch zu Mensch variieren. Bei diesem Verfahren werden die reflektierten und gestreuten Ultraschall-Wellen von Empfängern aufgenommen, so daß für eine genaue Identifikation oder Echtheitsbestimmung eine möglichst große Zahl von Empfängern notwendig ist. Da diese einen entsprechenden Platzbedarf haben, ist das Verfahren für ein schnelles Überprüfen der Echtheit beispielsweise der Epithelstruktur eines Fingers zum Beispiel in einem Geldausgabeautomat nicht geeignet. Wird die Zahl der Empfänger vermindert, um mit dem Platz auszukommen, können nicht mehr genügend Einzeldaten aufgenommen werden, um die Individualität der jeweiligen Epithelstruktur je nach aufgelegtem Finger mit Sicherheit erkennen und von einem anderen Finger unterscheiden zu können.

Es besteht daher die Aufgabe, ein Verfahren der eingangs erwähnten Art zu schaffen, womit auf engstem Raum eine hohe Auflösung einer solchen Struktur mit preiswerten Mitteln möglich ist.

Die Lösung der Aufgabe besteht darin, daß in dem Körper eine für die Ultraschallwellen durchlässige, elektromagnetische Wellen reflektierende und/oder beugende Schicht oder Fläche angebracht wird, daß diese Schicht oder Fläche in dem Körper durch die Ultraschallwellen je nach Form und Intensität der rückgestreuten oder gebeugten Ultraschallwellen lokal verschieden und zeitabhängig verformt wird, und daß diese Schicht mit elektromagnetischen Wellen unter einem Winkel bestrahlt wird und im Bereich der unter dem Ausfallswinkel reflektierten und/oder gebeugten elektromagnetischen Strahlen diese aufgefangen und ihre durch ihre Reflektion oder Beugung an der Schicht hervorgerufene Änderung ausgewertet wird.

Die Strukturen im Prüfobjekt insbesondere im Epithelgewebe des menschlichen Fingers verursachen bei entsprechend gewählter Ultraschall-Wellenlänge eine Beugung der auftreffenden Ultraschallwelle. Die gebeugte rücklaufende Ultraschallwelle ruft Verzerrungen bzw. Verformungen in dem Körper und auf der Schicht hervor. Da diese Schicht die Eigenschaft besitzt, elektromagnetische Wellen zu reflektieren oder zu beugen, können die auf der Schicht befindlichen Verformungsstrukturen optisch nachgewiesen werden. Es können z.B. als Detektoren sog. CCD-Arrays mit mehreren tausend matrixartig angeordneten Detektorelementen verwendet werden, was zu einer hohen Ortsauflösung und hoher Nachweisempfindlichkeit bei preisfünstig verfügbaren Detektoren führt.

Zweckmäßig ist es, wenn die Wellenlänge der Ultraschallquelle der Größe der zu identifizierenden Strukturen entsprechend gewählt wird. Dadurch tritt Beugung der Ultraschallwelle an den entsprechenden Strukturen auf.

Zweckmäßig ist es, wenn wenigstens ein Detektor zur Auswertung des reflektierten und/oder gebeugten elektromagnetischen Strahles, vorzugsweise des Lichtes vorgesehen wird und über eine gewählte Zeitdauer mißt.
Durch die Auswertung innerhalb eines Zeitfensters erhält man eine Momentaufnahme des reflektierten Strahls, durch Messung innerhalb von aufeinanderfolgenden, zeitlich getrennten Zeitfenstern erhält man einen zeitaufgelösten "Film" des sich dynamisch ändernden reflektierten Strahles.

Vorteilhaft ist es, wenn die reflektierende und/oder beugende Schicht im Strahlengang der Ultraschallwellen etwa senkrecht zu der Ausbreitungsrichtung der Ultraschallwellen angeordnet ist und die elektromagnetischen Wellen reflektierend am Übergang zwischen Körper und reflektierender Schicht eingestrahlt werden.
Indem die Ultraschall-Wellenfront die Schicht frontal passiert, vereinfacht sich das Meßverfahren. Durch die Reflektionseigenschaft der Schicht können optoelektronische Verfahren eingesetzt werden.

Zweckmäßig ist es, wenn die durch den Körper verlaufende Ultraschall-Bestrahlung des Prüfobjektes zeitweise unterbrochen wird, wobei die Unterbrechungen so bemessen werden, daß zumindest zeitweise Strahlen nur in der Sende- oder in der Reflektionsrichtung verlaufen Dadurch können die Messung störende Interferenzen zwischen einlaufender und rückgestreuter Ultraschallwelle vermieden werden.

Zweckmäßig ist es, wenn innerhalb eines Ultraschall-Pulses mehrere Ultraschallwellenlängen insbesondere zeitlich aufeinanderfolgend durchlaufen werden und wenn diese Wellenlängen auf die Größe der zu identifizierenden Strukturen abgestimmt werden. Dadurch lassen sich in der Größe stark voneinander abweichende Strukturen messen bzw. identifizieren.

Um die größtmögliche Ortsauflösung zu erzielen ist es zweckmäßig, wenn die reflektierten und/oder gebeugten elektromagnetischen Strahlen auf den/die Detektor(en) abgebildet werden. Damit das elektromagnetische Strahlenbündel die von mehreren Detektoren gebildete Fläche weder überstrahlt, wobei Information verlorenginge, noch die vorhandene Fläche unterstrahlt, wobei die größtmögliche Auflösung nicht erreicht würde, kann der Strahl mit einem Linsensystem angepaßt und auf die Detektoren abgebildet werden.

Eine zusätzliche Analysemöglichkeit ergibt sich, wenn je nach Verformung der reflektierenden und/oder beugenden Schicht an ihr auftreffendes weißes Licht spektral zerlegt wird.

Zur Auswertung ist es vorteilhaft, wenn die so gewonnenen Daten komprimiert oder bestimmte Daten der gesamten Datenmenge ausgewählt, kodiert und auf einem Datenträger gespeichert werden, der als Referenz für aktuelle Messungen dient. Das kann zur Bestimmung des jeweiligen Prüfobjektes verwendet werden, weil jeder durchstrahlte Körper verschiedene Strukturen haben kann, deren Auswirkungen auf die reflektierende Schicht allerdings um Größenordnungen geringer als die des zu identifizierenden Körpers sind. Vor allem können jedoch die von einem zu identifizierenden Körper gewonnenen individuellen Epithelstruktur-Daten auf einen Datenträger und/oder in einer Datenbank gespeichert werden, um jederzeit als Eich-Referenz zur Verfügung zu stehen, wenn die Person deren Finger untersucht wurde, identifiziert oder verifiziert werden muß, wie beispielsweise bei der Benutzung eines Geldausgabeautomaten.

Die Erfindung betrifft ferner eine Vorrichtung zur Durchführung des Verfahrens mit wenigstens einer Ultraschallquelle, mit einem von dieser durchstrahlbaren Körper und mit einer Auflagefläche für das Prüfobjekt, die in einem festen Winkel zum Strahlengang der Ultraschellquelle angeordnet ist.

Die erfindungsgemäße Vorrichtung ist dadurch gekennzeichnet, daß unterhalb der Auflagefläche für das Prüfobjekt eine elektromagnetische Wellen reflektierende und/oder beugende Schicht in dem Körper angeordnet ist und daß diese Vorrichtung eine Strahlenquelle für elektromagnetische Wellen, vorzugsweise eine Lichtquelle aufweist, deren Strahlen auf die Schicht gerichtet sind und daß im Bereich des reflektierten und/oder gebeugten Strahls wenigstens ein Strahlempfänger zum Auswerten der durch die Reflektion oder Beugung hervorgerufenen Änderungen dieses Strahls vorgesehen ist.

Durch die Reflektionseigenschaft kann ein der Schicht aufgeformtes momentanes Beugungsmuster optisch ausgelesen werden.

Zweckmäßig ist es, wenn die Schicht dicht unter der Auflagefläche etwa parallel zu dieser und etwa rechtwinklig zu dem Verlauf der Ultraschallwellen angeordnet ist.
Dadurch,daß die Ultraschall-Wellenfronten die Auflagefläche und die Schicht frontal passieren, vereinfacht sich das Meßverfahren.

Eine zweckmäßige Ausführungsform kann darin bestehen, daß die Schicht eine Spiegelschicht ist. Damit lassen sich besonders gute Reflektionseigenschaften erreichen. Gegebenenfalls können auch mehrere, vorzugsweise parallele reflektierende Flächen oder Schichten vorgesehen sein.

Zweckmäßig ist es, wenn mehrere Detektoren vorzugsweise in matrixähnlicher Weise angeordnet sind, wobei deren Flächennormale etwa parallel zum Ausfallswinkel des elektromagnetischen Strahls verläuft. Damit lassen sich preisgünstig erhältliche sog. CCD-Arrays mit hoher Ortsauflösung verwenden.

Zweckmäßig ist es, wenn die Ultraschallquelle mit wenigstens einem Impulsgeber oder dergleichen gekoppelt ist. Dadurch läßt sich die Ultraschallquelle pulsen. Die Abstände zwischen den aufeinanderfolgenden Pulsen sind so einzustellen, daß Interferenzen innerhalb des Körpers zwischen einlaufender und rückgestreuter Welle vermieden werden, die die Messung stören könnten.

Besonders vorteilhaft ist es, wenn wenigstens eine Projektionslinse oder dergleichen im Strahlengang der reflektierten und/oder gebeugten elektromagnetischen Strahlen zwischen dem Körper und dem/den Strahlempfänger(n) angeordnet ist. Der Strahl erhält dadurch einen an die vorhandene Detektorfläche angepaßten Strahldurchmesser. Durch diese Abbildung läßt sich die Ortsauflösung erhöhen.

Eine Fokussierung auf die Detektoren läßt sich erreichen, wenn die reflektierende und/oder beugende Schicht gewölbt ist. Die Wölbung kann wie ein Hohlspiegel wirken.

Nachstehend ist ein Ausführungsbeispiel der Erfindung mit ihren wesentlichen Einzelheiten anhand der Zeichnung noch näher beschrieben. Die einzelnen Merkmale können je für sich oder zu mehreren bei einer Ausführungsform der Erfindung verwirklicht sein.

Die einzige Figur zeigt in schematisierter Darstellung eine perspektivische Ansicht der Vorrichtung mit Körper und Prüfobjekt während einer Messung, wobei die im Körper befindliche reflektierende oder oder beugende Schicht oder Fläche entsprechend dem Beugungsmuster des Prüfobjektes dynamisch verformt ist.

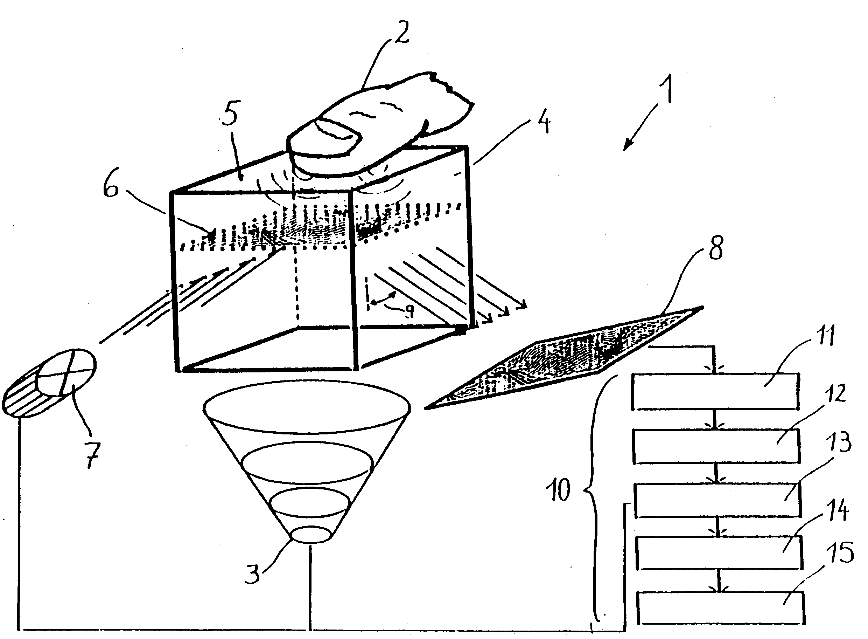

Eine in Fig.1 im ganzen mit 1 bezeichnete Vorrichtung dient zur Bestimmung der Echtheit und/oder Identifikation der jeweiligen Raumstruktur eines Prüfobjektes 2 - z.B. der Epithelstruktur der Haut, insbesondere einem menschlichen Fingers. Sie weist einen von einer Ultraschallquelle 3 durchstrahlbaren und für elektromagnetische Wellen durchlässigen Körper 4 auf, an dessen von der Ultraschallquelle 3 abgewandten Seite eine Auflagefläche 5 für das Prüfobjekt 2 angeordnet ist. Nahe unterhalb dieser Auflagefläche 5 ist innerhalb des Körpers 4 eine reflektierende oder beugende Schicht 6 angeordnet, die etwa parallel zur Auflagefläche 5 liegt und einen etwa rechten Winkel zu dem Verlauf der von der Ultraschallquelle 3 emittierten Ultraschallwellen bildet. Dabei sind der Körper 4 und die in ihm liegende Schicht 6, die beide für Ultraschallwellen durchlässig sind und in ihren elastischen Konstanten etwa gleich sind, um Streuverluste der Schallwellen an der Schicht 6 gering zu halten.

Die Vorrichtung 1 weist weiterhin eine elektromagnetische Strahlenquelle 7 - im Ausführungsbeispiel eine Lichtquelle - auf, deren Strahlen in den optisch transparenten Körper 4 seitlich eindringen und in diesem unter einem Winkel auf die Schicht 6 auftreffen. Da die Schicht 6 einen von dem Körper 4 verschiedenen Brechungsindex aufweist, werden die an der Schicht 6 auftreffenden elektromagnetischen Strahlen unter dem Ausfallswinkel 9 reflektiert, verlassen den Körper 4 und werden durch eine Detektoranordnung 8 aufgefangen, die mit einer Auswerteeinrichtung 10 verbunden ist.

Zur Messung der zu untersuchenden Struktur eines Prüfobjektes 2 insbesondere der Epithelstruktur der Haut z.B. des Fingers, wird der Finger auf die dafür vorgesehene Auflagefläche 5 des Körpers 4 aufgelegt. Die von der Ultraschallquelle 3 ausgesendeten Schallwellen durchlaufen den Körper 4, wobei die Wellenfronten die Schicht 6 etwa frontal passieren, und gelangen über die den Körper 4 begrenzende Auflagefläche 5 in die obersten Hautschichten des auf der Auflagefläche 5 aufliegenden Fingers.

Individuelle Strukturen des unter der äußersten Hautschicht liegenden Epithelgewebes haben Ausdehnungen, die in der Größenordnung der Wellenlänge von hochfrequentem Ultraschall liegen. Sie bewirken, daß die an innen auftreffenden Ultraschallwellen gebeugt und rückgestreut werden. Die in den Körper 4 zurücklaufende gebeugte Ultraschallwelle hat dort aufgrund der vom Epithelgewebe bewirkten Beugungseffekte lokal unterschiedliche Druckamplituden und verformt die Schicht 6 in dem Körper 4, wie in Fig.1 angedeutet ist, je nach Form und Intensität der zurücklaufenden gebeugten Ultraschallwelle lokal verschieden und zeitabhängig.

Da die Schicht 6 unter einem Winkel von der Lichtquelle bestrahlt wird, wobei die Schicht vorzugsweise eine Spiegelschicht ist, werden die auftreffenden elektromagnetischen Strahlen entsprechend den Verformungen, die die Schicht aufweist, in verschiedenen Winkeln mit unterschiedlicher Intensitätsverteilung unter dem Ausfallswinkel 9 reflektiert.

Im Bereich dieser Streu- oder Reflexionswinkel sind vorzugsweise mehrere Detektoren 8 insbesondere in matrixartiger Weise angeordnet, wobei die Flächennormale dieser Detektormatrix etwa parallel zum Ausfallswinkel 9 des Lichtstrahls verläuft. Das kann ein sog. "CCD-Array" (charge coupled device) mit mehreren tausend Detektorelementen sein, mit dessen Hilfe die durch die Reflektion hervorgerufenen lokalen Änderungen des Lichtstrahls gemessen werden. Damit der reflektierte Lichtstrahl die Detektorfläche 8 praktisch voll ausleuchtet um die größtmögliche Ortsauflösung zu erzielen, kann im Strahlengang des Lichtes zwischen dem Körper 4 und den Detektoren 8 eine Projectionslinse oder dergleichen angeordnet sein, die den reflektierten Strahl auf die Detektoren 8 entsprechend abbildet.

Eine vorteilhafte Ausführungsform der Vorrichtung 1 besteht darin, daß die durch den Körper 4 verlaufende Ultraschall-Bestrahlung des Prüfobjektes 2 periodisch unterbrochen wird, indem die Ultraschallquelle 3 mit einem - nicht dargestellten - Impulsgeber oder dergleichen gekoppelt wird. Die Unterbrechungsintervalle sind so bemessen, daß zumindest zeitweise die Ultraschallwellen nur in der einen oder der anderen, vom Prüfobjekt rückgestreuten Richtung laufen. Dadurch werden die Messung störende Interferenzen zwischen einlaufender und rücklaufender Ultraschallwelle praktisch ausgeschlossen. Um Beugungseffekte zu erzeugen, ist die Wellenlänge der Ultraschallquelle 3 der Größe der zu identifizierenden Strukturen entsprechend gewählt. Da jedoch unterschiedlich große Strukturen erfasst werden sollen, werden innerhalb eines Ultraschall-Pulses mehrere diskrete Ultraschall-Wellenlängen durchlaufen, wobei diese Wellenlängen etwa den Größen der unterschiedlichen Strukturen entsprechen. Dafür kann auch eine breitbandige Ultraschallquelle ( sog. "Transducer") eingesetzt werden.

Die Detektoranordnung 8, mit der die Intensitätsverteilung der einfallenden elektromagnetischen Strahlung gemessen wird, ist an eine im ganzen mit 10 bezeichneten Auswerte- und Steuerelektronik angeschlossen, wo die gemessenen Daten zwischengespeichert und nach den verschiedenen verwendeten Schallfrequenzen kodiert und auf einem Datenträger gespeichert werden. Die Auswertung beinhaltet bildverarbeitende Verfahren wie beispielsweise die Fouriertransformation. Die gespeicherten Daten stehen dann als Referenz für aktuelle Messungen zur Verfügung. Ein Steuerprozessor 13 ist mit der Ultraschallquelle 3 und deren Impulsgeber gekoppelt und bestimmt die Pulslängen, die Unterbrechungsintervalle und das Frequenzverhalten. Die Auswerte- und Steuerelektronik 10 besteht im Ausführungsbeispiel aus einer CCD-Schnittstelle 11, einer intelligenten Auswerte-Elektronik 12, einem Steuerprozessor 13, einem Krypto-Prozessor 14 zur Datencodierung sowie einem Schnittstellen-Modul 15 zur Vernetzung mit verschiedenen Datenbanken oder Terminals.

Eine eindeutige Identifikation bzw. Verifikation einzelner menschlicher Individuen ist möglich, da die Epithelstrukturen durch den speziellen genetischen Code und die individuellen Wachstumsphasen signifikant von Mensch zu Mensch variieren. Versuche haben gezeigt, daß alle Versuchspersonen mit diesem Verfahren anhand ihrer Haut z.B. am Finger eindeutig identifiziert und voneinander unterschieden werden konnten.

Zusammenfassend soll noch ausgeführt werden, daß das Meßverfahren auf diffraktiver Ortsauflösung individueller Strukturen eines Prüfobjektes 2 mittels Ultraschall beruht.
Da die Ultraschallwellen in der Größenordnung der aufzulösenden Strukturen oder etwas kleiner - wegen des hoben Kontrastes - gewählt werden, wird die rückgestreute Ultraschallwelle in verschiedene Beugungswinkel unterschiedlicher Intensität und Phasenlage gebeugt. Die gebeugte Ultraschallwelle verursacht dem Beugungsmuster entsprechend lokale, zeitabhängige Deformationen im Körper 4 und in der Schicht 6. Durch optische Reflektion an diesen zeitabhängigen Verformungen dieser Schicht 6 kann die zeitlich variierende Information optoelektronisch ausgelesen werden.

Der Körper 4 wird aus Glas bestimmter akustischer und optischer Qualität hergestellt. Statt eines Festkörpers können auch mit entsprechend gewählten Flüssigkeiten gefüllte Hohlkörper benutzt werden. In einem festen Körper 4 kann die Schicht 6 wie folgt angebracht werden. Der Körper 4 wird in einem Abstand etwa parallel zur Auflagefläche 5 aufgetrennt und die Schicht 6 kann beispielsweise durch photochemische oder epitaktische Verfahren angebracht oder aufgedampft werden. Danach kann der abgetrennte Teil des Körpers wieder mittels physikalisch-chemischer Verfahren auf diese Schicht 6 aufgebracht werden. Möglich ist aber auch die vollständige Herstellung des Körpers 4 mit der darin liegenden Schicht 6 durch epitaktische Methoden. Dadurch könnten auch materialspezifische Abweichungen zwischen diesen Körpern 4 gering gehalten werden.

## Patentansprüche

1. Verfahren zur Bestimmung der Echtheit und/oder Identifikation der jeweiligen Raumstruktur eines Prüfobjektes (2) insbesondere der Epithelstruktur der Haut z.B. eines menschlichen Fingers, wobei dieses Prüfobjekt (2) auf eine ebene oder gewölbte Fläche (5) aufgelegt und mit durch einen an diese Fläche (5) angrenzenden Körper (4) verlaufenden Ultraschallwellen beschallt wird und die am Prüfobjekt (2) reflektierten und/oder rückgestreuten Ultraschallwellen eine jeweils verschiedene Struktur unterschiedlicher Prüfobjekte (2) wiedergeben und ausgewertet werden, **dadurch gekennzeichnet**, daß in dem Körper (4) eine für die Ultraschallwellen durchlässige, elektromagnetische Wellen reflektierende und/oder beugende Schicht oder Fläche (6) angebracht wird, daß diese Schicht oder Fläche (6) in dem Körper (4) durch die Ultraschallwellen je nach Form und Intensität der rückgestreuten oder gebeugten Ultraschallwellen lokal verschieden und zeitabhängig verformt wird, und daß diese Schicht (6) mit elektromagnetischen Wellen unter einem Winkel bestrahlt wird und im Bereich der unter dem Ausfallswinkel (9) reflektierten und/oder gebeugten elektromagnetischen Strahlen diese aufgefangen und ihre durch ihre Reflektion oder Beugung an der Schicht (6) hervorgerufene Änderung ausgewertet werden.

2. Verfahren nach Anspruch 1, dadurch gekennzeichnet, daß die Wellenlänge der Ultraschallquelle (3) der Größe der zu identifizierenden Strukturen entsprechend gewählt wird.

3. Verfahren nach Anspruch 1 oder 2, dadurch gekennzeichnet, daß wenigstens ein Detektor (8) zur Auswertung des reflektierten und/oder gebeugten elektromagnetischen Strahles, vorzugsweise des Lichtes vorgesehen wird und über eine gewählte Zeitdauer mißt.

4. Verfahren nach einem der Ansprüche 1 bis 3, dadurch gekennzeichnet, daß die reflektierende und/oder beugende Schicht (6) im Strahlengang der Ultraschallwellen etwa senkrecht zu der Ausbreitungsrichtung der Ultraschallwellen angeordnet ist und die elektromagnetischen Wellen reflektierend am Übergang zwischen Körper (4) und reflektierender Schicht (6) eingestrahlt werden.

5. Verfahren nach einem der Ansprüche 1 bis 4, dadurch gekennzeichnet, daß die durch den Körper (4) verlaufende Ultraschall-Bestrahlung des Prüfobjektes (2) zeitweise unterbrochen wird, wobei die Unterbrechungen so bemessen werden, daß zumindest zeitweise Strahlen nur in der Sende- oder in der Reflektionsrichtung verlaufen.

6. Verfahren nach einem der Ansprüche 1 bis 5, dadurch gekennzeichnet, daß innerhalb eines Ultraschall-Pulses mehrere Ultraschallwellenlängen insbesondere zeitlich aufeinanderfolgend durchlaufen werden und daß diese Wellenlängen auf die Größe der zu identifizierenden Strukturen abgestimmt werden.

7. Verfahren nach einem der Ansprüche 1 bis 6, dadurch gekennzeichnet, daß die reflektierten und/oder gebeugten elektromagnetischen Strahlen auf den/die Detektor(en) (8) abgebildet werden.

8. Verfahren nach einem der Ansprüche 1 bis 7, dadurch gekennzeichnet, daß je nach Verformung der reflektierenden und/oder beugenden Schicht (6) an ihr auftreffendes weißes Licht spektral zerlegt wird.

9. Verfahren nach einem der Ansprüche 1 bis 8, dadurch gekennzeichnet, daß die so gewonnenen Daten komprimiert oder bestimmte Daten der gesamten Datenmenge ausgewählt, kodiert und aus einem Datenträger gespeichert werden, der als Referenz für aktuelle Messungen dient.

10. Vorrichtung zur Durchführung des Verfahrens nach einem oder mehreren der vorstehenden Ansprüche mit wenigstens einer Ultraschallquelle (3), mit einem von dieser durchstrahlbaren Körper (4) und mit einer Auflagefläche (5) für das Prüfobjekt (2) an dem durchstrahlbaren Körper (4), die in einem festen Winkel zum Strahlengang der Ultraschallquelle (3) angeordnet ist, **dadurch gekennzeichnet**, daß unterhalb der Auflagefläche (5) für das Prüfobjekt (2) eine elektromagnetische Wellen reflektierende und/oder beugende durch Ultraschallwellen verformbare Schicht (6) in dem Körper (4) angeordnet ist und daß die Vorrichtung (1) eine Strahlenquelle (7) für elektromagnetische Wellen, vorzugsweise eine Lichtquelle aufweist, deren Strahlen auf die Schicht (6) gerichtet sind und daß im Bereich des reflektierten und/oder gebeugten elektromagnetischen Strahles wenigstens ein Strahlempfänger (8) zum Auswerten der durch die Reflektion oder Beugung hervorgerufenen Änderungen dieses Strahls vorgesehen ist.

11. Vorrichtung nach Anspruch 10, dadurch gekennzeichnet, daß die Schicht (6) dicht unter der Auflagefläche (5) etwa parallel zu dieser und etwa rechtwinklig zu dem Verlauf der Ultraschallwellen angeordnet ist.

12. Vorrichtung nach Anspruch 10 oder 11, dadurch gekennzeichnet, daß die Schicht (6) eine Spiegelschicht ist.

13. Vorrichtung nach einem der Ansprüche 10 bis 12, dadurch gekennzeichnet, daß mehrere, vorzugsweise parallele reflektierende oder beugende Flächen oder Schichten (6) vorgesehen sind.

14. Vorrichtung nach einem der Ansprüche 10 bis 13, dadurch gekennzeichnet, daß mehrere Detektoren (8) vorzugsweise in matrixähnlicher Weise angeordnet sind, wobei deren Flächennormale etwa parallel zum Ausfallswinkel (9) des elektromagnetischen Strahls verläuft.

15. Vorrichtung nach einem der Ansprüche 10 bis 14, dadurch gekennzeichnet, daß die Ultraschallquelle (3) mit wenigstens einem Impulsgeber oder dergleichen gekoppelt ist.

16. Vorrichtung nach einem der Ansprüche 10 bis 15, dadurch gekennzeichnet, daß wenigstens eine Projektionslinse oder dergleichen im Strahlengang der reflektierten und/oder gebeugten elektromagnetischen Strahlen zwischen dem Körper (4) und dem/den Strahlempfänger(n) (8) angeordnet ist.

17. Vorrichtung nach einem der Ansprüche 10 bis 16, dadurch gekennzeichnet, daß die reflektierende und/oder beugende Schicht (6) gewölbt ist.

## Claims

1. A method for determining the authenticity and/or for identifying the respective spatial structure of an object (2) to be inspected, particularly the epithelial structure of the skin e.g. of a human finger, whereby said object (2) to be inspected is placed on a plane or curved surface (5) and is exposed to ultrasonic waves traversing a body (4) contiguous with said surface (5) and the ultrasonic waves reflected and/or backscattered at the object (2) reproduce a different structure in each case of different objects (2) and are evaluated, characterized in that provided in the body (4) is a layer or surface (6) which is transparent to the ultrasonic waves and reflects and/or diffracts electromagnetic waves, that said layer or surface (6) in the body (4) is deformed in a locally different and time-dependent way by the ultrasonic waves according to the form and intensity of the backscattered or diffracted ultrasonic waves, and that said layer (6) is exposed to radiation of electromagnetic waves at an angle, and in the area of the electromagnetic beams reflected and/or diffracted at the angle of emergence (9) the same are intercepted and their change caused by their reflection or diffraction at the layer (6) is evaluated.

2. A method as claimed in claim 1, characterized in that the wavelength of the ultrasonic source (3) is selected according to the size of the structures to be identified.

3. A method as claimed in claim 1 or claim 2, characterized in that at least one detector (8) is provided for evaluation of the reflected and/or diffracted electromagnetic beam, preferably of the light, and measures over a selected duration.

4. A method as claimed in any one of claims 1 to 3, characterized in that the reflecting and/or diffractive layer (6) in the beam path of the ultrasonic waves is arranged approximately perpendicular to the propagation direction of the ultrasonic waves and the electromagnetic waves are reflectively irradiated at the transition between body (4) and reflecting layer (6).

5. A method as claimed in any one of claims 1 to 4, characterized in that the exposure of the object (2) to ultrasonic waves traversing the body (4) is periodically interrupted, the interruptions being determined in such a way that, at least intermittently, beams travel only in the direction of transmission or in the direction of reflection.

6. A method as claimed in any one of claims 1 to 5, characterized in that within an ultrasonic pulse a plurality of ultrasonic wavelengths are traversed, particularly consecutively in time and that said wavelengths are coordinated to the size of the structures to be identified.

7. A method as claimed in any one of claims 1 to 6, characterized in that the reflected and/or diffracted electromagnetic beams are imaged on the detector(s) (8).

8. A method as claimed in any one of claims 1 to 7, characterized in that, depending on the deformation of the reflecting and/or diffractive layer (6), white light impinging thereon is spectrally decomposed.

9. A method as claimed in any one of claims 1 to 8, characterized in that the data thus gained are compressed or certain data of the entire dataset are selected, coded and stored on a data carrier serving as reference for current measurements.

10. An apparatus for carrying out the method as claimed in one or several of the preceding claims, including at least one ultrasonic source (3), a body (4) penetrable to radiation thereby and on the latter (4) a supporting surface (5) for the object (2), said supporting surface being arranged at a fixed angle to the beam path of the ultrasonic source (3), characterized in that arranged in the body (4), beneath the supporting surface (5) for the object (2), is a layer (6) deformable by ultrasonic waves and reflecting and/or diffracting electromagnetic waves, and that said apparatus (1) has a radiation source (7) for electromagnetic waves, preferably a light source, the beams of which are directed to the layer (6) and that at least one beam receiver (8) is provided in the area of the reflected and/or diffracted electromagnetic beam and serves to evaluate the changes of said beam caused by the reflection or diffraction.

11. An apparatus as claimed in claim 10, characterized in that the layer (6) is arranged closely beneath and approximately parallel to the supporting surface (5) and approximately at right angles to the path of the ultrasonic waves.

12. An apparatus as claimed in claim 10 or claim 11, characterized in that the layer (6) is a mirror layer.

13. An apparatus as claimed in any one of claims 10 to 12, characterized in that a plurality of preferably parallel, reflecting or diffracting surfaces or layers (6) are provided.

14. An apparatus as claimed in any one of claims 10 to 13, characterized in that a plurality of detectors (8) are arranged preferably in a matrix-like manner, the normal to the surface thereof running approximately parallel to the emergence angle (9) of the electromagnetic beam.

15. An apparatus as claimed in any one of claims 10 to 14, characterized in that the ultrasonic source (3) is coupled to at least one pulser or the like.

16. An apparatus as claimed in any one of claims 10 to 15, characterized in that at least one projector lens or the like is arranged in the beam path of the reflected and/or diffracted electromagnetic beams between the body (4) and the beam receiver(s) (8).

17. An apparatus as claimed in any one of claims 10 to 16, characterized in that the reflecting and/or diffracting layer (6) is curved.

## Revendications

1. Procédé pour déterminer l'authenticité et/ou pour identifier la structure respective dans l'espace d'un objet à contrôler (2), notamment la structure épithéliale de la peau d'un doigt humain par exemple, cet objet à contrôler (2) étant posé sur une surface plane ou bombée (5) et étant exposé à l'action d'ondes ultrasonores traversant un corps (4) limitrophe de cette surface (5), et les ondes ultrasonores réfléchies et/ou rétrodiffusées sur l'objet à contrôler (2) reproduisant une structure chaque fois différente pour différents objets à contrôler (2), et étant évalueés, **caractérisé** en ce qu'une couche ou surface (6), laissant passer les ondes ultrasonores et réfléchissante et/ou diffractante pour les ondes électromagnétiques, est placée dans le corps (4), en ce que cette couche ou surface (6) dans le corps (4) est déformée par les ondes ultrasonores en fonction du temps et de façon localement différente selon la forme et l'intensité des ondes ultrasonores rétrodiffusées ou diffractées, et en ce que cette couche (6) est irradiée sous un angle par des ondes électromagnétiques et, dans la région des rayons électromagnétiques diffractés et/ou réfléchis sous l'angle de réflexion (9), on recueille ces rayons et on évalue leur modification produite par leur réflexion ou diffraction sur la couche (6).

2. Procédé selon la revendication 1, **caractérisé** en ce que la longueur d'onde de la source ultrasonore (3) est choisie conformément à la taille des structures à identifier.

3. Procédé selon la revendication 1 ou 2, **caractérisé** en ce qu'au moins un détecteur (8) est prévu pour l'évaluation du rayon électromagnétique réfléchi et/ou diffracté, de préférence de la lumière, et effectue la mesure pendant une durée choisie.

4. Procédé selon une des revendications 1 à 3, **caractérisé** en ce que la couche réfléchissante et/ou diffractante (6) est disposée dans le trajet des rayons des ondes ultrasonores environ perpendiculairement à la direction de propagation des ondes ultrasonores, et les ondes électromagnétiques sont irradiées de façon à exercer une action réfléchissante à la transition entre le corps (4) et la couche réfléchissante (6).

5. Procédé selon une des revendications 1 à 4, **caractérisé** en ce que l'exposition aux rayons ultrasonores de l'objet à contrôler (2) à travers le corps (4) est périodiquement interrompue, les interruptions étant dimensionnées de façon qu'au moins périodiquement, des rayons se propagent uniquement dans la direction d'émission ou dans la direction de réflexion.

6. Procédé selon une des revendications 1 à 5, **caractérisé** en ce que plusieurs longueurs d'ondes ultrasonores sont parcoutues, notamment successivement dans le temps, à l'intérieur d'une impulsion ultrasonore, et en ce que ces longueurs d'ondes sont adaptées à la taille des structures à identifier.

7. Procédé selon une des revendications 1 à 6, **caractérisé** en ce que les rayons électromagnétiques réfléchis et/ou diffractés sont projetés sur le ou les détecteurs (8).

8. Procédé selon une des revendications 1 à 7, **caractérisé** en ce que, selon la déformation de la couche réfléchissante et/ou diffractante (6), on décompose le spectre de la lumière blanche qui l'atteint.

9. Procédé selon une des revendications 1 à 8, **caractérisé** en ce qu'on comprime les données ainsi obtenues ou bien on sélectionne des données déterminées dans la quantité totale de données, on les code et on les mémorise sur un support de données, qui sert de référence pour les mesures actuelles.

10. Dispositif pour la mise en oeuvre du procédé selon une ou plusieurs des revendications précédentes, avec au moins une source ultrasonore (3), avec un corps (4) à travers lequel peut rayonner cette source et avec une surface d'appui (5) pour l'objet à contrôler (2) sur le corps (4) laissant passer le rayonnement, surface qui est disposée sous un angle fixe par rapport au trajet des rayons de la source ultrasonore (3), **caractérisé** en ce qu'une couche (6), réfléchissante et/ou diffractante pour les ondes électromagnétiques et pouvant être déformée par les ondes ultrasonores, est disposée dans le corps (4) en dessous de la surface d'appui (5) pour l'objet à contrôler (2) et en ce que le dispositif (1) présente une source de rayonnement (7) pour des ondes électromagnétiques, de préférence une source lumineuse, dont les rayons sont dirigés sur la couche (6), et en ce qu'au moins un récepteur de rayonnement (8) est prévu dans la région du rayon électromagnétique réfléchi et/ou diffracté, afin d'évaluer les modifications de ce rayon qui sont produites par la réflexion au diffraction.

11. Dispositif selon la revendication 10, **caractérisé** en ce que la couche (6) est disposée juste en dessous de la surface d'appui (5), environ parallèlement à cette dernière et environ perpendiculairement à l'allure des ondes ultrasonores.

12. Dispositif selon la revendication 10 ou 11, **caractérisé** en ce que la couche (6) est une couche de miroir.

13. Dispositif selon une des revendications 10 à 12, **caractérisé** en ce qu'on prévoit plusieurs couches ou surfaces réfléchissantes ou diffractantes (6), de préférence parallèles.

14. Dispositif selon une des revendications 10 à 13, **caractérisé** en ce que plusieurs détecteurs (8) sont disposés de préférence à la manière d'une matrice, la normale à leur surface s'étendant environ parallèlement à l'angle de réflexion (9) du rayon électromagnétique.

15. Dispositif selon une des revendications 10 à 14, **caractérisé** en ce que la source ultrasonore (3) est couplée à au moins un générateur d'impulsions ou analogue.

16. Dispositif selon une des revendications 10 à 15, **caractérisé** en ce qu'au moins une lentille de projection ou analogue est disposée dans le trajet des rayons électromagnétiques réfléchis et/ou diffractés, entre le corps (4) et le ou les récepteurs de rayonnement (8).

17. Dispositif selon une des revendications 10 à 16, **caractérisé** en ce que la couche réfléchissante et/ou diffractante (6) est bombée.
